# EUROPEAN PATENT APPLICATION

(11) **EP 4 191 603 A1**
(43) Date of publication of application: **07.06.2023**
(21) Application number: 22157468.4
(22) Date of filing: 18.02.2022
(51) Int. Cl.: G16H 40/20, G16H 10/60, G06N 20/00, G06Q 10/00

(54) **PATIENT MESSAGING TO REDUCE NO-SHOWS USING DATA CAPTURED VIA PATIENT ENGAGEMENT PLATFORM**

(30) Priority: 02.12.2021 US 202163285273 P
(71) Applicant: Koninklijke Philips N.V., 5656 AG Eindhoven (NL)
(72) Inventor: RAGHAVAN, Ushanandini, Eindhoven (NL); MONFORTE, Anthony, Eindhoven (NL); SINGH, Rohit, Eindhoven (NL)
(74) Representative: Philips Intellectual Property & Standards

(57) **Abstract**

A non-transitory computer readable medium (26) stores a schedule (32) of patient appointments; and instructions executable by at least one electronic processor to perform a patient appointment notification method (100) including, for a patient having a patient appointment on the schedule: providing a messaging system via which patient appointment notifications are pushed to the patient; converting data related to the patient and the patient appointment into features; analyzing the features to determine a score (40) indicative of likelihood of the patient no-showing the appointment; adjusting, based on the score, at least one of (i) times at which the messaging system pushes patient appointment notifications (50) to the patient and/or (ii) content of the patient appointment notifications pushed to the patient and/or (iii) an interactivity of the patient appointment notifications pushed to the patient.

## Description

### FIELD OF THE INVENTION

The following relates generally to the medical arts, patient appointment notification arts, risk model prediction arts, mobile application arts, and related arts.

### BACKGROUND OF THE INVENTION

Patients often do not show up (i.e., "no show") for medical appointments, either without any notice or cancelling at a time close to an appointment (i.e., a "late cancellation"). No shows by patients to medical appointments have significant ramifications for both the hospital and the patients. Unused patient appointment time slots and medical device underutilization negatively impact a medical facility's finances and physician's ability to provide effective and timely clinical care. Missed appointments can also negatively impact patient outcomes. No shows for departments like primary care can result in increased emergency department (ED) admissions and a reduction in quality of life for patients.

At the same time, backlogs of to-be-scheduled appointments continue to grow. If appointments such as screenings do not happen within a certain timeframe, reimbursement rates, hospital ratings, and clinical outcomes are also negatively impacted. Supplemental and/or automated processes to assist in growing workloads would assist support staff and to keep patients engaged in their care.

The following discloses new and improved systems and methods to overcome these problems and others.

### SUMMARY OF THE INVENTION

In one aspect, a non-transitory computer readable medium stores a schedule of patient appointments; and instructions executable by at least one electronic processor to perform a patient appointment notification method including, for a patient having a patient appointment on the schedule: providing a messaging system via which patient appointment notifications are pushed to the patient; converting data related to the patient and the patient appointment into features; analyzing the features to determine a score indicative of likelihood of the patient no-showing the appointment; adjusting, based on the score, at least one of (i) times at which the messaging system pushes patient appointment notifications to the patient and/or (ii) content of the patient appointment notifications pushed to the patient and/or (iii) an interactivity of the patient appointment notifications pushed to the patient.

In another aspect, a non-transitory computer readable medium stores instructions executable by at least one electronic processor to perform a patient appointment scheduling method including converting data related to a patient and an appointment scheduled for the patient into features; analyzing the features to determine likelihood of the patient no-showing the appointment; generating a score indicative of the likelihood of the patient no-showing the appointment; and outputting, on an electronic processing device, the score.

In another aspect, a patient appointment notification method includes, for a patient having a patient appointment on a schedule: providing a messaging system via which patient appointment notifications are pushed to the patient; applying a machine learning (ML) component to analyze features of data related to the patient and the patient appointment to determine a score indicative of likelihood of the patient no-showing the appointment; and outputting, on an electronic processing device, the score.

One advantage resides in reducing patient no shows without notice or with late cancellation.

Another advantage resides in efficiently allocating medical facility resources for patient appointments.

Another advantage resides in reducing patient appointment backlogs.

Another advantage resides in providing notifications to patients for upcoming appointments.

Another advantage resides in implementing a risk prediction model to aid healthcare providers in estimating the likelihood of an appointment being wasted because of a no-show without notice or with late cancellation, leading to decisions about whether to double-book or overbook appointment slots.

Another advantage resides in estimating how many patients are likely to miss appointments, leading to decisions on interventions that can prevent patients from no-showing.

Another advantage resides in using patient-specific data in order to estimate a likelihood of a no show without notice or with late cancellation of an appointment by the patient.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
Fig. 1 diagrammatically shows an illustrative apparatus for generating patient appointment notifications in accordance with the present disclosure.
Figs. 2 and 3 show an example flow chart of operations suitably performed by the apparatus of Fig. 1.
Fig. 4 shows example notifications generated by the apparatus of Fig. 1.

### DETAILED DESCRIPTION OF EMBODIMENTS

The following relates to a system for predicting no-shows or late cancellations (which, as used herein, are considered to be synonymous). Data are collected including "tenant" data (e.g., encompassing patient data, insurance data, and the like in possession of the client hospital) and external data such as patient zip code. The data are converted to features for analysis by machine learning (ML) to estimate likelihoods of no shows. For example, zip code data for the hospital and patient are converted to a travel distance.

In analyses of historical patient no show occurrences underlying certain embodiments disclosed herein, certain features were observed to be notably useful in predicting no shows, including patient past behavior; feedback from message engagement (e.g. did the patient respond to a text message reminder of the appointment); and type of appointment (e.g., an appointment with a nurse is more likely to result in a no show than an appointment with a doctor).

ML algorithms employing an ensemble of models, such as XgBoost and Random Forest, were found in tests to be effective in predicting patient no-shows. However, different optimal models may be suitably used for different clients (e.g. hospitals serving communities with different demographic profiles), and in some embodiments a different model is contemplated to be trained for each client.

In some embodiments, the trained ML model is applied to generate an N day ahead score of likelihood of a no show for each patient. Patients scoring a high likelihood of being a no show have their touchpoints adjusted. A "touchpoint" as used herein refers to an instance of contacting the patient to remind or encourage the patient to show up for the appointment. Touchpoints have associated times of delivery, and may be in the form of text messages, emails, robotic telephone calls, or so forth. A patient scoring a high likelihood of being a no show may be assigned more touchpoints, and/or the touchpoints may be adjusted to be more active (e.g. encouraging or requiring patient interaction such as selecting a confirmation button). Responses to the touchpoints (or lack thereof) provide feedback features that are applied for subsequent N-day ahead scoring. For example, if the 7-day ahead score is computed before any touchpoints have been executed, then a 6-day ahead score will not be calculated, and in another example, if a 5-day ahead score is calculated, this score will include a 6-day ahead score.

The disclosed system in some embodiments includes the trained ML no show likelihood scoring in combination with a messaging system executing the touchpoints linked by feedback from the touchpoint results back to subsequent ML no show likelihood scoring and consequent modification of the touchpoints.

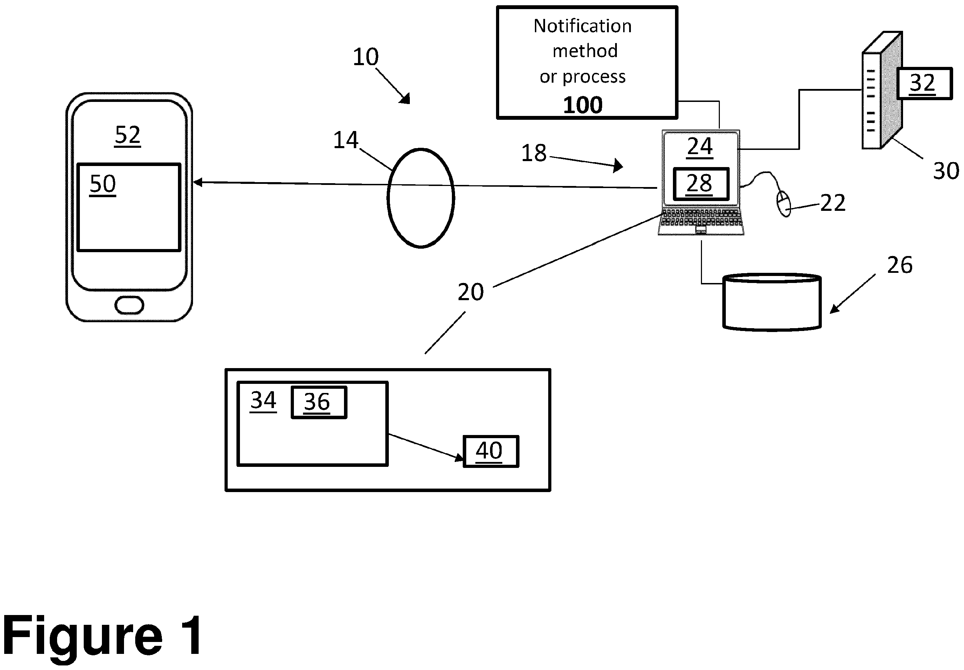

With reference to Fig. 1, an illustrative apparatus 10 is shown for providing patient appointment notifications to patients. Fig. 1 also shows an electronic processing device 18, such as a workstation computer, or more generally a computer. The electronic processing device 18 typically includes or accesses a patient scheduling system, and may also include a server computer or a plurality of server computers, e.g., interconnected to form a server cluster, cloud computing resource, or so forth, to perform more complex computational tasks. The workstation 18 includes typical components, such as an electronic processor 20 (e.g., a microprocessor), at least one user input device (e.g., a mouse, a keyboard, a trackball, and/or the like) 22, and a display device 24 (e.g., an LCD display, plasma display, cathode ray tube display, and/or so forth). In some embodiments, the display device 24 can be a separate component from the workstation 18, or may include two or more display devices.

The electronic processor 20 is operatively connected with one or more non-transitory storage media 26. The non-transitory storage media 26 may, by way of non-limiting illustrative example, include one or more of a magnetic disk, RAID, or other magnetic storage medium; a solid-state drive, flash drive, electronically erasable read-only memory (EEROM) or other electronic memory; an optical disk or other optical storage; various combinations thereof; or so forth; and may be for example a network storage, an internal hard drive of the workstation 18, various combinations thereof, or so forth. It is to be understood that any reference to a non-transitory medium or media 26 herein is to be broadly construed as encompassing a single medium or multiple media of the same or different types. Likewise, the electronic processor 20 may be embodied as a single electronic processor or as two or more electronic processors. The non-transitory storage media 26 stores instructions executable by the at least one electronic processor 20. The instructions include instructions to generate a visualization of a graphical user interface (GUI) 28 for display on the display device 24.

The apparatus 10 also includes, or is otherwise in operable communication with, one or more database(s) 30 storing a data related to patients with scheduled medical procedures having appointment times, and/or data related to operations of the medical facility where the scheduled medical procedures are to occur. The database 30 can be any suitable database, including a Radiology Information System (RIS) database, an Electronic Medical Records (EMR) database, an Electronic Health Records (EHR) database, a Cardiology Information System (CIS) database, a Health-Level 7 (HL7)-compliant database, and so forth. Physically, the database 30 can be implemented in the non-transitory medium or media 26. In addition, the database(s) 30 can include a public database storing residence address information for the patients, along with weather information and local traffic information based on the address information of the patient. The residence address information can include, for example, a zip code of the patients, a street address of the patient, or both.

In general, the one or more database(s) 30 include or provide the functionality of: (i) a scheduler 32, which maintains a schedule of appointments for the medical examinations; and (ii) a patient data repository such as an EMR or EHR that stores patient data from which the patient features are extracted. As an example of the scheduler 32, a radiology laboratory may have a scheduler component built into the RIS which provides for making patient radiology examination appointments as well as storing the physician-issued radiology examination order for each patient. More generally the scheduler could be maintained for another hospital department, e.g. histopathology, oncology, or so forth, and/or for an individual physician or physician group or other medical servicing entity. The scheduler 32 typically stores specific appointment times for the patients having scheduled appointments.

Fig. 1 also shows a messaging system including modules implemented by the at least one electronic processor 20 to generate and send patient appointment notifications. The illustrative messaging system advantageously is configured to tailor the timing and content of appointment notifications based on estimates for individual patients of their likelihoods of no showing for scheduled appointments. A machine-learning (ML) component 34 (e.g., an XgBoost model, a Random Forest model, or any other suitable ML algorithm) is configured to generate a score 40 indicative of a likelihood that the patient will no show without notice or with late cancellation of the medical appointment. To do so, the ML component 34 is configured by training on historical no show data to implement a prediction model 36 to determine which patients are likely to no show for their appointments. In this regard, a no show is considered to be any instance in which a patient fails to show for the appointment without notice, or provides notice with insufficient lead time so as to be considered to be a late cancellation. The deadline after which a cancellation is deemed to be a late cancellation may depend on the medical organization. For example, some medical institutions deem cancellation less than 24 hours before an appointment to be a late cancellation. In general, historical no show training data for training the ML component 34 suitably labels historical no shows including late cancellations based on the chosen deadline for defining a late cancellation. A no show is thus considered to be any instance in which the patient fails to show for the appointment without notification or provides late cancellation.

Based on the score 40 for a particular patient, the at least one electronic processor 20 is programmed to adjust the timing and/or content of a notification 50 and transmit the notification 50 to a mobile device 52 of the patient via a communication link 14, which typically comprises the Internet possibly augmented by local area networks and/or cellular network(s). For example, the patient can log-in into a mobile application program ("app") which is provided as a component of the patient engagement system, and is loaded on, and executable on, the mobile device 52 of the patient (e.g., an illustrative cellular telephone 52, or a tablet computer, personal data assistant or PDA, and/or so forth) to receive the notification 50. In a suitable embodiment, the app is represented on the home screen or applications screen of the mobile device 52 as an app icon (i.e., a small square, round, or other compact graphical element representing the app) and the user launches (i.e., initiates running of) an instance of the app on the device 52 by touching the icon on a (touch-sensitive) screen of the mobile device 52. In one example, in lieu of the app, the notification 50 can be sent to the mobile device 52 in any suitable format, such as a pre-recorded voice communication, an automated phone call, an email, a short message service (SMS) message, (i.e., a text message), and so forth.

The apparatus 10 is configured as described above to perform a patient appointment reminder method or process 100 for a patient having a patient appointment on the schedule 32. The non-transitory storage medium 26 stores instructions which are readable and executable by the at least one electronic processor 20 to perform disclosed operations including performing the patient appointment notification method or process 100. In some examples, the method 100 may be performed at least in part by cloud processing.

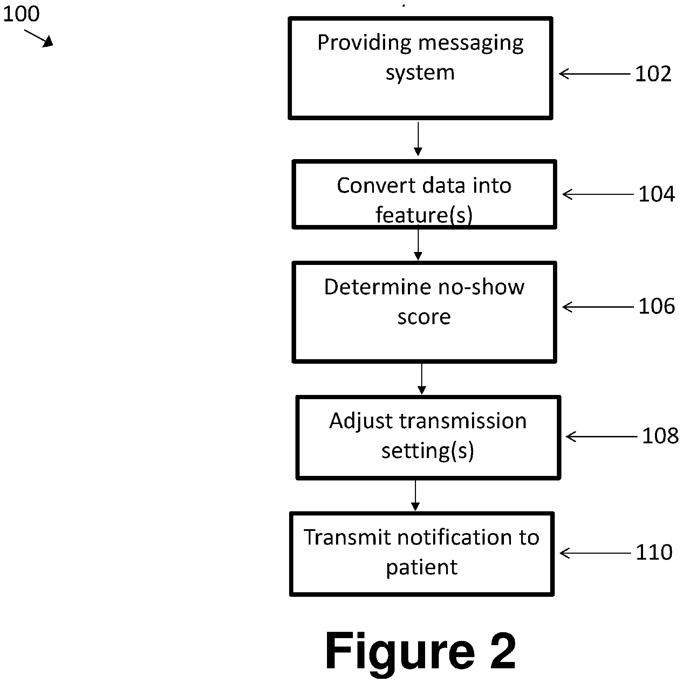

With reference to Fig. 2, an illustrative embodiment of an instance of the patient appointment notification method 100 is diagrammatically shown as a flowchart. The method 100 can begin with an operation 102, which includes providing a messaging system via which patient appointment reminders are pushed to a patient having a patient appointment on the schedule 32. The messaging system can comprise the electronic processing device 18 pushing the notifications 50 to mobile device 52 of the patient via the communication link 14.

At an operation 104, data related to the patient and the patient appointment are converted into one or more features. The data can include one or more of patient health record data, insurance data, patient location data, patient past behavior data, patient messaging feedback data, and type of appointment.

At an operation 106, the features are analyzed to determine the score 40 indicative of likelihood of the patient no-showing the appointment. To do so, in some embodiments, the ML component 34 is applied to the features to determine the score 40. The score 40 can be, for example, scored on a scale of 0 and 1 in which a score of 0 is indicative of a low risk of no show and a score of 1 is indicative of a high risk of no show. (This is just an example, and other scoring scales can be used, such as a percentage of a likelihood of a no-show; it is also contemplated to rescale and/or discretize the score, e.g., to an integer value between 0 and 10).

In some embodiments, the score generating operation 106 can include generating the score 40 as an N-day ahead score of a likelihood of a patient no-showing the appointment. The N-day score 40 is indicative of a number of days before the appointment before the notification 50 about the appointment is sent to the patient. To calculate the N-day score 40, a number of times at which the messaging system pushes patient appointment notifications 50 to the patient indicative of instances of contacting the patient to remind the patient about the appointment are calculated. The N-day score 40 is generated from the number of calculated times at which the messaging system pushes patient appointment notifications 50.

At an operation 108, one or more settings of transmission of the notifications 50 to the mobile device 52 of the patient can be adjusted based on the score 40. In some embodiments, times at which the messaging system pushes the patient appointment notifications 50 to the patient can be adjusted. In other embodiments, content of the patient appointment notifications 50 can be adjusted based on the score 40. In further embodiments, an interactivity of the patient appointment notifications 50 can be adjusted based on the score 40. An interactivity of the patient appointment notifications 50 can include, for example, options such as "No Response Requested," "Confirmation Requested" (e.g., "Will you make this appointment?" with "Yes" or "No" selection buttons, but no enforcement if no response), "Confirmation required" (e.g., "The appointment will be canceled if you do not confirm at least 24 hours ahead of the appointment"), and so forth. These are merely examples and should not be construed as limiting.

At an operation 110, the notification 50 is transferred to the mobile device 52 of the patient can be adjusted based on the score 40. In some embodiments, an acknowledgment from the patient responsive to the notification 50 is received, and the N-day ahead score 40 for the patient can be reduced in response to receiving the acknowledgment confirming the appointment. In another example, an acknowledgment confirming the appointment is received without adjusting the score 40 for the patient. In a further example, the N-day score 40 for the patient can be increased. These are merely examples and should not be construed as limiting.

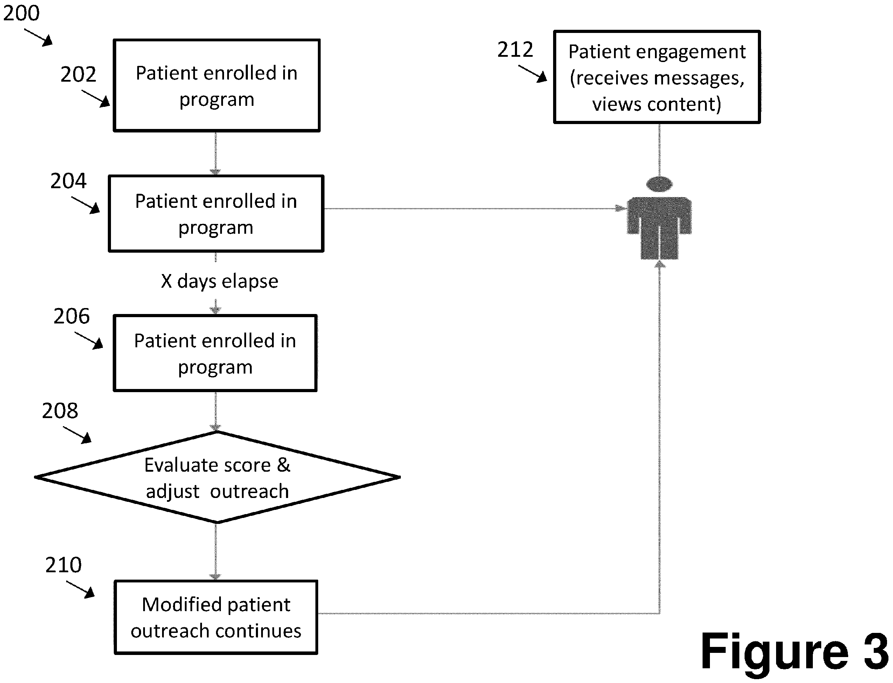

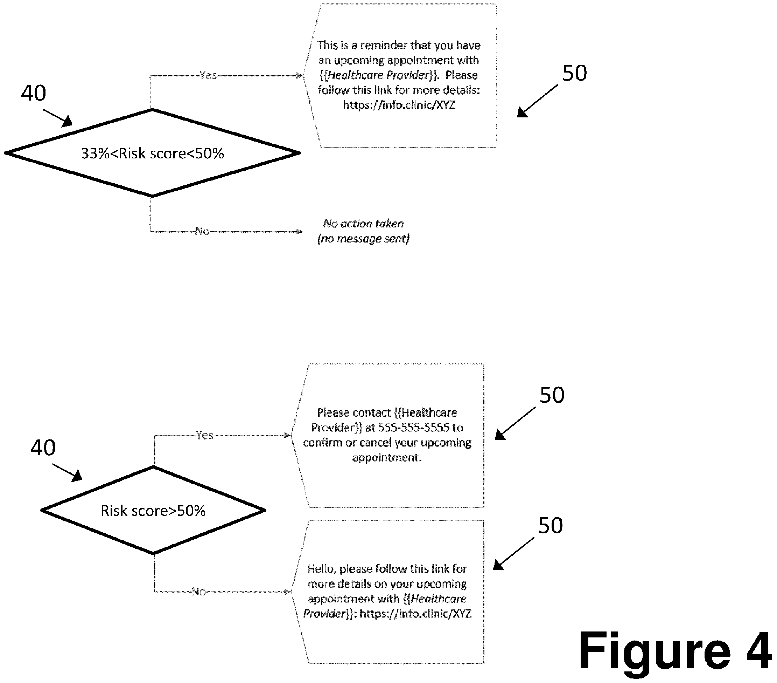

Fig. 3 shows another embodiments of the method 200. At an operation 202, a patient is enrolled in the messaging system. This may entail acquiring and storing scheduling and demographic data. At an operation 204, a patient outreach (e.g., a text message, an email, and so forth) is begun. At an operation 206, the predictive model 36 is used to generate the score 40, e.g. for Y days prior to the appointment. At an operation 208, the score 40 is evaluated and used to adjust the outreach to the patient, e.g. by modifying notification content and/or timing and/or interactivity. At an operation 210, a modified patient outreach is performed (e.g. via SMS, email etc.), via which the patient receives the notifications 50 (designated as operation 212). FIGURE 4 shows examples of the notifications 50 based on the scores 40.

### EXAMPLE

The following describes an example of generating the risk model 36. Data (e.g., patient health record data, insurance data, patient location data, patient past behavior data, patient messaging feedback data, type of appointment, and so forth) are stored in a standard database (such as a Microsoft SQL DB). Different data sets are linked via patient IDs and are then merged to create one big file for each patient appointment. Tenant data includes outcomes (no show / cancel / show), demographics such as Age, Gender, Marital Status etc, Insurance providers, patient IDs and appointment dates to generate longitudinal data and features. External data includes, for example, zip code, latitude, and longitude data to calculate a distance between patient zip code and appointment zip code. In addition, data that can provide information on federal holidays, state holidays, and disease / procedure categorization codes can also be stored. All raw data are converted into features that are then fed as input to the ML component 34.

The data set is then divided into multiple training and test sets. Machine learning algorithms such as standard logistic regression, Lasso regression, Ridge regression, Random Forest, XgBoost and Support Vector Machines (see, e.g., https://towardsdatascience.com/10-machine-learning-methods-that-every-data-scientist-should-know-3cc96e0eeee9) are used to train models of the ML component 34. Among these, the model that generates highest accuracy on the test set is chosen, which an accuracy is calculated as F1-score (see, e.g., https://en.wikipedia.org/wiki/F-score).

Events related to the appointment can be captured. Such events can include, for example, patient registration (e.g., either registering themselves, registering a co-pilot, registered by a healthcare provider etc.) to the messaging system, interaction (click on a static link in the email and/or text message sent to the patient, click a module sent via text and/or email etc.), and options (such as whether they want to opt out, whether they were reachable via phone and/or email). A miscellaneous category can be included that, among others, captures if the phone used by the patient is an iPhone or Android or others etc.

The disclosure has been described with reference to the preferred embodiments. Modifications and alterations may occur to others upon reading and understanding the preceding detailed description. It is intended that the disclosure be construed as including all such modifications and alterations insofar as they come within the scope of the appended claims or the equivalents thereof.

## Claims

1. A non-transitory computer readable medium (26) storing:
a schedule (32) of patient appointments; and
instructions executable by at least one electronic processor (20) to perform a patient appointment notification method (100), the method comprising, for a patient having a patient appointment on the schedule:
providing a messaging system via which patient appointment notifications are pushed to the patient;
converting data related to the patient and the patient appointment into features;
analyzing the features to determine a score (40) indicative of likelihood of the patient no-showing the appointment;
adjusting, based on the score, at least one of (i) times at which the messaging system pushes patient appointment notifications (50) to the patient and/or (ii) content of the patient appointment notifications pushed to the patient and/or (iii) an interactivity of the patient appointment notifications pushed to the patient.

2. The non-transitory computer readable medium (26) of claim 1, wherein the method (100) comprises:
adjusting, based on the score (40), times at which the messaging system pushes patient appointment notifications (50) to the patient.

3. The non-transitory computer readable medium (26) of either one of claims 1 and 2, wherein the method (100) comprises:
adjusting, based on the score (40), content of the patient appointment notifications (50) pushed to the patient.

4. The non-transitory computer readable medium (26) of any one of claims 1-3, wherein the method (100) comprises:
adjusting, based on the score (40), an interactivity of the patient appointment notifications (50) pushed to the patient.

5. The non-transitory computer readable medium (26) of any one of claims 1-4, wherein analyzing the features to determine likelihood of a patient no-showing the appointment includes:
applying a machine learning (ML) component (34) to the features to determine the likelihood of the patient no-showing the appointment.

6. The non-transitory computer readable medium (26) of claim 5, wherein the ML component (34) comprises an XgBoost model or a Random Forest model.

7. The non-transitory computer readable medium (26) of any one of claims 1-6, wherein the data includes one or more of patient health record data, insurance data, patient location data, patient past behavior data, patient messaging feedback data, and type of appointment.

8. The non-transitory computer readable medium (26) of any one of claims 1-7, wherein generating a score (40) indicative of the likelihood of a patient no-showing the appointment includes:
generating an N-day score of a likelihood of a patient no-showing the appointment, the N-day score is indicative of a number of days before the appointment before a notification about the appointment is sent to the patient.

9. The non-transitory computer readable medium (26) of claim 8, wherein generating the N-day score (40) includes:
calculating a number of times at which the messaging system pushes patient appointment notifications (50) to the patient indicative of instances of contacting the patient to remind the patient about the appointment; and
generating the N-day score from the number of calculated times at which the messaging system pushes patient appointment notifications.

10. The non-transitory computer readable medium (26) of claim 9, wherein the method (100) comprises:
adjusting, based on the score (40), the times at which the messaging system pushes patient appointment notifications (50) to the patient.

11. The non-transitory computer readable medium (26) of any one of claims 8-10, wherein the method (100) further includes:
transmitting a notification (50) to a patient electronic device (52) about the appointment based on the generated N-day score (40).

12. The non-transitory computer readable medium (26) of claim 11, wherein the method (100) further includes one of:
receiving an acknowledgment from the patient confirming the appointment responsive to the notification (50) and reducing the N-day score (40) for the patient in response to receiving the acknowledgment confirming the appointment; or
receiving no indication of acknowledgment from the patient responsive to the notification; or
increasing the N-day score for the patient.

13. A non-transitory computer readable medium (26) storing instructions executable by at least one electronic processor (20) to perform a patient appointment scheduling method (100), the method comprising:
converting data related to a patient and an appointment scheduled for the patient into features;
analyzing the features to determine likelihood of the patient no-showing the appointment;
generating a score (40) indicative of the likelihood of the patient no-showing the appointment; and
outputting, on an electronic processing device (52), the score.

14. The non-transitory computer readable medium (26) of claim 13, wherein the method (100) comprises:
adjusting, based on the score (40), at least one of (i) times at which the messaging system pushes patient appointment notifications (50) to the patient and/or (ii) content of the patient appointment notifications pushed to the patient and/or (iii) an interactivity of the patient appointment notifications pushed to the patient.

15. The non-transitory computer readable medium (26) of either one of claims 13 and 14, wherein analyzing the features to determine likelihood of a patient no-showing the appointment includes:
applying a machine learning (ML) component (34) to the features to determine the likelihood of the patient no-showing the appointment.

16. The non-transitory computer readable medium (26) of any one of claims 13-15, wherein generating a score (40) indicative of the likelihood of a patient no-showing the appointment includes:
calculating a number of times at which a messaging system pushes patient appointment notifications (50) to the patient indicative of instances of contacting the patient to remind the patient about the appointment; and
generating an N-day ahead score from the number of calculated times at which the messaging system pushes patient appointment notifications, the day ahead score is indicative of a number of days before the appointment before a notification about the appointment is sent to the patient.

17. The non-transitory computer readable medium (26) of claim 16, wherein the method (100) comprises:
adjusting, based on the score (40), the times at which the messaging system pushes patient appointment notifications (50) to the patient.

18. The non-transitory computer readable medium (26) of either one of claims 16 and 17, wherein the method (100) further includes:
transmitting a notification (XX) to a patient electronic device (XX) about the appointment based on the generated N-day ahead score (XX).

19. The non-transitory computer readable medium (26) of claim 18, wherein the method (100) further includes one of:
receiving an acknowledgment from the patient confirming the appointment responsive to the notification (50) and reducing the N-day ahead score (40) for the patient in response to receiving the acknowledgment confirming the appointment; or
receiving no indication of acknowledgment from the patient responsive to the notification; or
increasing the N-day ahead score for the patient.

20. A patient appointment notification method (100) comprising, for a patient having a patient appointment on a schedule (32):
providing a messaging system via which patient appointment notifications are pushed to the patient;
applying a machine learning (ML) component (34) to analyze features of data related to the patient and the patient appointment to determine a score (40) indicative of likelihood of the patient no-showing the appointment; and
outputting, on an electronic processing device (52), the score.
